# EUROPEAN PATENT APPLICATION

(11) **EP 1 800 680 A1**
(43) Date of publication of application: **27.06.2007**
(21) Application number: 05793118.0
(22) Date of filing: 11.10.2005
(51) Int. Cl.: A61K 31/4172, A61K 45/00, A61P 9/10

(54) **CEREBRAL INFARCTION SUPPRESSANT**

(30) Priority: 15.10.2004 JP 2004301991; 15.03.2005 JP 2005073941
(71) Applicant: Ehime University, Matsuyama-shi, Ehime 790-8577 (JP); Okayama University, Okayama-shi, Okayama 700-0082 (JP)
(72) Inventor: ADACHI, Naoto,Mabuchi Clinic, Shimogyo-ku, Kyoto-shi, Kyoto 600-8357 (JP); LIU, Keyue, Touon-shi, Ehime 791-0295 (JP); MOTOKI, Atsuko, Touon-shi, Ehime 791-0295 (JP); SEMBA, Kazunori, Touon-shi, Ehime 791-0295 (JP); HIRAGA, Norihito, Touon-shi, Ehime 791-0295 (JP); NISHIBORI, Masahiro,Graduate School of Medicine, Okayama-shi (JP)
(74) Representative: Gille Hrabal Struck Neidlein Prop Roos
(86) International application number: PCT/JP2005/018702
(87) International publication number: WO 2006/041061

(57) **Abstract**

An object of the present invention is to provide a cerebral infarction suppressant which is effective for brain tissue necrosis attributed to long-time ischemia as in actual cerebral infarction and has fewer side effects. The suppressant for cerebral infarction attributed to long-time ischemia according to the present invention is **characterized in that** a histidine and an H₃-receptor blocker or a histidine and a histamine N-methyltransferase inhibitor are comprised as active ingredients.

## Description

### TECHNICAL FIELD

The present invention relates to an agent for suppressing cerebral infarction attributed to cerebral ischemia.

### BACKGROUND ART

Cerebral infarction is a disease wherein the cerebral blood vessel is occluded or narrowed due to various factors, such as transportation of thrombus formed in an extracerebral blood vessel into the brain and arteriosclerosis of the cerebral blood vessel. These result in insufficient blood flow in the brain and necrosis of tissue with impaired blood flow. Once cerebral infarction occurs, the brain tissue which has developed necrosis never regain its function. Therefore, even if the patient survives, symptoms of dementia, motor weakness, sensory abnormality and language disorder often persist. On the other hand, in recent years, diseases called lifestyle-related diseases, such as hypertension, cardiac diseases, hyperlipidemia and diabetes, are increasing, and the risks for cerebral infarction are increasing. Therefore, an effective method for treating cerebral infarction is earnestly desired.

However, a truly effective treatment has not been found yet until now. For example, a thrombolytic agent is used in order to recover blood flow by removing a thrombus that provokes cerebral infarction. However, since disorders by free radicals generated after recovery of blood flow are also closely related to cerebral damage, the thrombolytic agent alone cannot provide the fundamental solution to prevent necrosis of the brain tissue.

Additionally, edaravone, i.e. a free radical scavenger to protect brain tissues from free radicals that cause the impairment of the brain tissue, is commercially available. However, the agent has side effects such as hepatic and renal damage. In particular, 21.4% of patients who received the agent showed abnormal values in laboratory tests on the liver function. Even though cerebral infarction may be life-threatening, the treatment with such a high incidence of side effects is problematic.

Hypothermic therapy is one of the treatments besides medication. However, in addition to high costs required for the treatment, infection resulting from lowered immunity and bleeding tendency make general application of the treatment difficult.

Even if brain cells do not develop necrosis, it is known that the programmed death, i.e. apoptosis, of neurons is induced by brief period of ischemia, i.e. transient ischemia. For example, it is described by Kawamoto Toshiki et. al. in "Protective effect of L-histidine (singlet oxygen scavenger) on transient forebrain ischemia in rat", Brain and Nerve, 49(7), pp.612-618 (1997) that loss of pyramidal neurons in the hippocampal CA-1 region was observed one week after 5 or 10 minutes of transient forebrain ischemia in rats. This is called "delayed neuronal death". The main cause of the apoptosis by transient ischemia is considered to be elevated extracellular concentration of glutamic acid, which is an excitatory amino acid. Namely, due to an increase in the concentration of glutamic acid, the intracellular concentration of calcium ion increases, and expression of genes that cause cell death and biochemical reactions is induced. In the above document, there is a description that the programmed death is depressed by administration of L-histidine prior to transient ischemia, and a decrease in the concentration of glutamic acid is described as one of the reasons.

Also, it is described by Naoto Adachi et. al. in "Alleviation of ischemic neuronal damage by postischemic loading with histidine in the rat striatum", Brain Research, 998, pp.136-138 (2004) that neuronal death 7 days after ischemia caused by apoptosis was depressed in rats by administration of histidine 4 times: shortly, 6 hours, 24 hours and 48 hours after loading with 15 minutes of transient ischemia. However, since the concentration of glutamic acid caused by transient ischemia is supposed to have already started when histidine is administered after ischemia, neuronal death is presumably inhibited by another mechanism different from that by pre-ischemic administration.

In the literature, there is a description that the alleviation of nerve cell death by histidine is slightly suppressed by mepyramine, which is an H₁-receptor blocker, and further disappears completely by using ranitidine concomitantly, which is an H₂-receptor blocker. Therefore, according to the disclosure of the literature, when histidine is applied to nerve cell death attributed to transient ischemia, it is important not to use a histamine receptor blocker at the same time.

In spite of these well-known findings, a method for suppressing neuronal death by transient ischemia cannot necessarily be applied to actual cerebral infarction. This is due to the complexity of factors inducing cell death and the difference in the preventive mechanism of neuronal death between pre- and post-ischemic administration as described above. Additionally, the actual cause of cerebral infarction, i.e. brain tissue necrosis, is prolonged cerebral ischemia for several hours, of which mechanism is different from that of delayed neuronal death occurring 7 days after a few to a dozen minutes of ischemia. For example, though apoptosis caused by transient ischemia develops only in neurons, necrosis caused by prolonged ischemia develops not only in neurons but also in all brain tissues including glial cells and vascular endothelial cells.

Considering the actual treatment for cerebral infarction, it is impossible to begin the therapy only several minutes after the onset of thrombosis or embolism; it is usually several hours after the onset. Further, while the primary therapy is the recovery of blood flow by removing the thrombus as soon as possible, it is extremely important to prevent the development of necrosis in brain tissues caused by reperfusion injury to a minimum, since neurons which once died never recover their function. The treatment for apoptosis, which will progress later, should be of second importance, and effective treatments to inhibit apoptosis are not always effective for suppression of necrosis.

### DISCLOSURE OF THE INVENTION

As described above, some research findings are released on apoptosis of neurons, which develops a few days after brief ischemia, due partly to academic interests. However, the findings are not always applicable to necrosis of brain tissues directly caused by prolonged ischemia. On the other hand, a treatment method against necrosis of brain tissues subsequent to prolonged ischemia is truly desired, since no effective method for cerebral infarction is available.

An object of the present invention is to provide a cerebral infarction suppressant effective against brain tissue necrosis after prolonged ischemia corresponding to actual cerebral infarction.

The present inventors have carried out extensive examinations on agents capable of suppressing brain tissue necrosis after ischemia for several hours. As a result, in spite of the finding by Naoto Adachi et. al. that histamine receptor blocker has a negative effect on suppressive effects of histidine on delayed nerve cell death attributed to short-time ischemia, the present inventors have found that necrosis can be considerably suppressed by a use of histidine and H₃-receptor blocker together. Further, the present inventors have also found that an excellent result can be obtained by a concomitant use of histidine and histamine N-methyltransferase inhibitor, and accomplished the present invention.

A first cerebral infarction suppressant of the present invention is characterized in that a histidine and an H₃-receptor blocker are comprised as active ingredients. A second cerebral infarction suppressant of the present invention is characterized in that a histidine and a histamine N-methyltransferase inhibitor are comprised as active ingredients.

A use according to the present invention is characterized in that a histidine and an H₃-receptor blocker, or a histidine and a histamine N-methyltransferase inhibitor are used for manufacturing a therapeutic agent for cerebral infarction.

A method of treatment for cerebral infarction according to the present invention is characterized in that a histidine and an H₃-receptor blocker, or a histidine and a histamine N-methyltransferase inhibitor are administered.

### BEST MODE FOR CARRYING-OUT OF THE INVENTION

In general, cerebral infarction is caused by ischemia resulting from brain thrombosis, cerebral embolism and the like, and accompanied by morphological damage, i.e. necrosis, which is large enough to be visible with the naked eye. On the other hand, in apoptosis resulting from brief ischemia, e.g. for a few to a dozen minutes, by a temporary decrease in cerebral blood flow, a small thrombus and the like, neuronal loss occurs in a few days in a restricted site vulnerable to ischemia. Even if apoptosis provokes any symptoms, the symptoms are much more moderate than those in cerebral infarction, and do not threaten life. Further, generating mechanisms for necrosis and apoptosis are clearly different. The cerebral infarction suppressant of the present invention is targeted to cerebral infarction caused by long-time ischemia.

The "long-time" in prolonged ischemia is not particularly limited, but it is at least the period during which necrosis of brain tissues is directly induced by ischemia. Specific time depends on the cause or the degree of ischemia, the difference in individual or the like. Considering a duration from the onset of ischemia to the start of the actual treatment, the specific time may be, for example, 1 hour or more, more preferably 1.5 hours or more, even more preferably 2 hours or more.

The cerebral infarction suppressant of the present invention comprises histidine as one of the active ingredients. Since histidine is one of the essential amino acids, it is considered to have fewer side effects and can be administered at high doses. Further, histidine readily crosses the blood-brain barrier, and is converted to histamine in the brain by decarboxylase. The histamine is considered to act on the brain tissue.

A first cerebral infarction suppressant of the present invention suppresses cerebral infarction synergistically by comprising a histidine and an H₃-receptor blocker concomitantly as active ingredients. The H₃-receptor exists in the presynaptic region of histaminergic nerves, wherein histamine plays a role as a neurotransmitter, and regulates release of histamine from nerve endings and synthesis of histamine from histidine. The release of histamine is suppressed by stimulating the H₃-receptor. Namely, an H₃-receptor controls feedback regulation of histaminergic nerves. Accordingly, the cerebral infarction suppressant of the present invention is considered to promote synthesis of histamine from histidine in the brain and release of histamine for a long time and thus enhance the effects of histamine further by inhibiting the feedback regulation by using the H₃-receptor blocker.

Examples of the H₃-receptor blocker used in the present invention may include thioperamide, clobenpropit, GT-2016, AQ-0145 and FUB181, and thioperamide is preferably used. The effects of thioperamide have been proven in the aftermentioned Examples.

A second cerebral infarction suppressant of the present invention suppresses cerebral infarction by comprising a histidine and a histamine N-methyltransferase inhibitor (hereinafter referred to as "HMT inhibitor") concomitantly as active ingredients. Histidine is converted to histamine in the brain as described above, and the histamine is further methylated by an enzyme called histamine N-methyltransferase, i.e. HMT, to be metabolized. Therefore, the present invention has succeeded in maintaining the effects of histamine by using histidine and an HMT inhibitor concomitantly.

Examples of the HMT inhibitor used in the present invention may include metoprine and SKF91488, i.e. Carbamimidothioic acid, and metoprine is preferably used. The effects of metoprine have been proven in the aftermentioned Examples.

The cerebral infarction suppressant of the present invention may comprise three drugs of a histidine, an H₃-receptor blocker and an HMT inhibitor as active ingredients besides the agents described above. Simultaneous administration of three drugs shows high effects particularly, even when the dose of histidine is reduced as described in the aftermentioned examples. In the embodiment, thioperamide as the H₃-receptor blocker and metoprine as the HMT inhibitor are preferred.

The formulation of the cerebral infarction suppressant according to the present invention includes an agent containing all of the active ingredients, and a kit consisting of two or three agents each of which contains at least one active ingredient. Namely, the cerebral infarction suppressant of the present invention may be an agent containing all the active ingredients of a histidine and an H₃-receptor blocker; a histidine and an HMT inhibitor; and a histidine, an H₃-receptor blocker and an HMT inhibitor. Alternatively, the suppressant of the present invention may be a kit consisting of two or more agents, for example, a kit consisting of an agent containing a histidine and an agent containing an H₃-receptor blocker. In a case of a kit, each agent may be administered concomitantly or successively with some intervals at a rate preferred by users or under doctor's supervision. Among these formulations, a formulation containing all of the active ingredients is preferred. It is because, in addition to the convenience during production, the synergistic effects of these active ingredients are provided firmly and quickly.

The cerebral infarction suppressant according to the present invention is preferably administered during ischemia or after ischemia, though the suppressant may be administered in a preventive manner before ischemia. Namely, the cerebral infarction suppressant of the present invention is administered after the incidence of long-time ischemia concomitantly with the administration of a thrombolytic drug or after reperfusion.

Although the optimum time point to administer the cerebral infarction suppressant of the present invention is not particularly limited, the suppressant is preferably administered during ischemia-reperfusion or after ischemia-reperfusion. There is not a clear distinction between "during ischemia-reperfusion" and "after ischemia-reperfusion" in "during ischemia-reperfusion or after ischemia-reperfusion", and "during ischemia-reperfusion or after ischemia-reperfusion" refers to, for example, before and after a certain treatment for ischemia-reperfusion such as administration of a thrombolytic drug, simultaneously with the treatment or predetermined time after the treatment. At least, administration before ischemia is not included in "during ischemia-reperfusion or after ischemia-reperfusion". When the suppressant is administered before ischemia, the suppressant is substantially deemed to be a preventive agent, and administration before ischemia is impossible in the case of cerebral infarction due to its sudden and unexpected onset.

The cerebral infarction suppressant of the present invention is preferably administered after reperfusion. This is because it is considered to be important for suppressing cerebral infarction to prevent restenosis of blood vessels after reperfusion by enhancing the histamine activity in the brain tissue, and suppress necrosis of brain tissue attributed to reperfusion injury to a minimum by administering cerebral infarction suppressant of the present invention after reperfusion. More preferably, the suppressant is administered shortly after reperfusion. This "shortly after" does not strictly mean shortly after reperfusion, but, for example, within 30 minutes after a certain treatment for ischemia-reperfusion is employed such as administration of a thrombolytic drug.

The form and route of administration of the suppressant according to the present invention are not particularly limited. In view of urgency of cerebral ischemia, intravenous administration as an injection is preferred. In such a case, pH-adjusted physiological saline, pure water, distilled water, sterile water, or the like may be used as a solvent.

As shown in the Examples described later, prominent effects on suppressing cerebral infarction were observed in the case where 1000 mg/kg of histidine and 5 mg/kg of H₃-receptor blocker were administered, 1000 mg/kg of histidine and 10mg/kg of HMT inhibitor were administered, and 500 mg/kg of histidine, 5 mg/kg of H₃-receptor blocker and 10 mg/kg of HMT inhibitor were administered to a rat weighing about 300 g. Considering the results, doses of histidine, the H₃-receptor blocker and the HMT inhibitor for humans are estimated to be 50 to 500 mg/kg, 0.1 to 2 mg/kg and 0.1 to 2 mg/kg per dosage, respectively. In particular, when using the three drugs concomitantly, it is possible to reduce the dose of histidine. However, the doses of these drugs should be suitably changed depending on patient's age, sex and severity of illness and the like. Continuous administration by drip infusion should also be considered.

Additionally, the cerebral infarction suppressant of the present invention is preferably administered for a plurality of times or in a continuous manner. Even when blood flow is resumed, restenosis of blood vessels, which is closely associated with inflammatory cell infiltration, such as neutrophils and the like, often develops after long-time occlusion of cerebral blood vessels. Therefore, in treating cerebral infarction, the concentration of histamine in the brain tissue needs to be maintained at a high level over a prolonged period of time, to prevent vascular restenosis and inflammatory responses. Specifically, it is administered preferably during ischemia-reperfusion or shortly after ischemia-reperfusion and once again 4 to 8 hours thereafter, more preferably during ischemia-reperfusion or shortly after ischemia-reperfusion and twice or more every 4 to 8 hours thereafter as a total of 3 times or more. Further, when the suppressant is administered in a continuous manner, each dose is preferably administered over one to several hours by drip infusion and the like.

The present invention will be explained more specifically by examples below. However, the present invention is not limited by the following examples, and necessary alterations can be made on it to an extent applicable to the above-described and later-described points. All of them are included in the technical scope of the present invention.

### EXAMPLES

### Example 1: Experiment of concomitant use of histidine and H₃-receptor blocker

Forty-two male Wistar rats weighing about 300 g were divided into 6 groups consisting of histidine 200 mg/kg administration group, histidine 500 mg/kg administration group, histidine 1000 mg/kg administration group, histidine 200 mg/kg + thioperamide 5 mg/kg administration group, histidine 500 mg/kg + thioperamide 5mg/kg administration group, and histidine 1000 mg/kg + thioperamide 5 mg/kg administration group. These rats were anesthetized with a gas mixture of 2% halothane, 49% oxygen and 49% laughing gas, and kept under spontaneous breathing. Subsequently, a median incision was made in the neck of the rat placed on its back, and the right common carotid artery was exposed. After an intraperitoneal injection of 100 units of heparin, the root of the right middle cerebral artery was occluded by inserting 4·0 nylon thread coated with silicone into the right internal carotid artery from the bifurcation of the internal and external carotid arteries. The tip of the nylon thread was placed 18 mm from the bifurcation. After suture of the incision of the skin, the rats were allowed to recover from anesthesia. During surgery, an electronic thermometer was inserted into the temporal muscle, and the body temperature was maintained at 37.0±0.1°C with a lamp. After recovery from anesthesia, paralysis of the contralateral limb was observed in all rats.

Five minutes before reperfusion of blood flow, the rats were anesthetized again. After opening the skin suture, cerebral blood flow was resumed by removing the nylon thread by 5 mm 2 hours after middle cerebral artery occlusion. Then, the incision was sutured again, and 200 mg/kg, 500 mg/kg or 1000 mg/kg of histidine was administered intraperitoneally. Histidine was first dissolved in physiological saline adjusted to pH 4.0 with hydrochloric acid, and then the pH was resumed to 6.0 with sodium hydroxide. Further, 5 mg/kg of thioperamide was intraperitoneally injected to each thioperamide administration group. After recovery from anesthesia, the rats were allowed to access food and water freely.

Twenty-four hours after recovery of blood flow, sodium pentobarbital was intraperitoneally administered to the rats for anesthesia. Subsequently, the brain was perfused with physiological saline, and the rat was decapitated. The brain was quickly dissected, and rinsed in physiological saline. The brain was sliced coronally between the optic chiasm and the caucal edge of the mammillary body at a thickness of 2 mm. These brain slices were subjected to incubation with 2% triphenyltetrazolium chloride in phosphate buffer (0.1 mol/L, pH 7.4) at 37°C. As a result, triphenyltetrazolium chloride was reduced due to the action of dehydrogenase present in viable cells, and the tissue was stained in dark red. On the other hand, the dead tissue in the infarcted area was not stained. These brain slices were preserved in phosphate-buffered formalin overnight. Then, an investigator who was unaware of histidine administration measured the size of infarction using computer-aided planimetry. The obtained size of cerebral infarction was subjected to analysis of variance and the Scheffe test. The results are shown in Table 1.

**Table 1**

| Histidine | Thioperamide | | Striatum | Cerebral cortex | Total |
|---|---|---|---|---|---|
| 200 mg/kg | - | n=6 | 35.3±21.6 | 106.3±63.0 | 141.6±83.1 |
| 200 mg/kg | 5 mg/kg | n=7 | 34.4±16.0 | 81.4±26.0 | 115.8±39.2 |
| 500 mg/kg | - | n=6 | 30.5±23.3 | 86.0±49.9 | 116.5±69.7 |
| 500 mg/kg | 5 mg/kg | n=9 | 22.3±15.8 | 77.0±35.1 | 99.3±42.1 |
| 1000 mg/kg | - | n=6 | 47.8±16.3 | 114.2±33.9 | 162.0±45.8 |
| 1000 mg/kg | 5 mg/kg | n=8 | 18.4±27.0 | 24.6±35.1* | 43.1±61.8* |

The values (unit: mm3) in the table indicate the size of cerebral infarction as the "mean ± standard deviation", while "*" denotes a case in which the value was significant versus the corresponding group without thioperamide administration by p<0.05.

According to the results, in the groups without thioperamide administration, suppressive effects on cerebral infarction were not found by administration of histidine alone in a single dose after ischemia. On the other hand, with respect to the groups treated with histidine and thioperamide concomitantly, the size of cerebral infarction in the 1000 mg/kg histidine + thioperamide 5 mg/kg administration group was reduced to about 27% of the group without thioperamide administration, i.e. the group treated with histidine alone.

From the above results, it was demonstrated that a concomitant use of histidine and H₃-receptor blocker has an effect of suppressing cerebral infarction.

### Example 2: Experiment of concomitant use of histidine and HMT inhibitor

Twenty-five male Wistar rats weighing about 300 g were divided into 4 groups consisting of histidine 1000 mg/kg administration group, histidine 200 mg/kg + metoprine 10 mg/kg administration group, histidine 500 mg/kg + metoprine 10 mg/kg administration group, and histidine 1000 mg/kg + metoprine 10 mg/kg administration group.

The root of the right middle cerebral artery of these rats was occluded and blood flow was recovered as shown in the Example 1. Then, 200 mg/kg, 500 mg/kg or 1000 mg/kg of histidine was intraperitoneally administered to the rats, and 10 mg/kg of metoprine was further administered to the metoprine administration groups, as shown in the Example 1.

The samples were prepared by treating each rat as shown in the Example 1 and the size of infarction was measured. The obtained size of cerebral infarction was subjected to analysis of variance and the Bonferroni test. The results are shown in Table 2.

**Table 2**

| Histidine | Metoprine | | Striatum | Cerebral cortex | Total |
|---|---|---|---|---|---|
| 1000 mg/kg | - | n=6 | 57.2±20.2 | 87.8+42.5 | 145.0±59.2 |
| 200 mg/kg | 10 mg/kg | n=6 | 48.6±26.0 | 84.9±60.1 | 133.4±84.4 |
| 500 mg/kg | 10 mg/kg | n=6 | 52.1±29.6 | 85.9±64.3 | 138.0±90.1 |
| 1000 mg/kg | 10 mg/kg | n=7 | 7.6±16.2** | 7.1±15.6* | 14.8±31.4* |

Each value in the table is shown as that in the Example 1, and "*" denotes a case in which the value was significant versus that of the group without metoprine administration, i.e. the group treated with histidine alone by p<0.05, while "**" denotes a case in which the value was significant by p<0.01.

According to the results, in the group without metoprine administration, suppressive effects on cerebral infarction were not found only by administering 1000 mg/kg of histidine in a single dose after ischemia. On the other hand, with respect to the group treated with histidine and metoprine concomitantly, the size of cerebral infarction in the histidine 1000 mg/kg + metoprine 10mg/kg administration group was reduced to about 10% of that of the group without metoprine administration, i.e. the group treated with histidine alone.

As described above, suppressive effects of a concomitant use of histidine and an HMT inhibitor on cerebral infarction was demonstrated.

### Example 3: Experiment of concomitant use of histidine, H₃-receptor blocker and HMT inhibitor

Eighteen male Wistar rats weighing about 300 g were divided into 3 groups consisting of metoprine 10 mg/kg + thioperamide 5 mg/kg administration group, histidine 200 mg/kg + metoprine 10 mg/kg + thioperamide 5 mg/kg administration group, and histidine 500 mg/kg + metoprine 10 mg/kg + thioperamide 5 mg/kg administration group. The experiment was carried out as shown in the Examples 1 and 2, and the results of measurement were subjected to analysis of variance and Bonferroni test. The results are shown in Table 3.

**Table 3**

| Histidine | Thioperamide | Metoprine | | Striatum | Cerebral cortex | Total |
|---|---|---|---|---|---|---|
| - | 5mg/kg | 10mg/kg | n=6 | 40.9±15.6 | 73.5±40.9 | 114.3±52.6 |
| 200mg/kg | 5mg/kg | 10mg/kg | n=6 | 52.7±9.6 | 68.6±24.3 | 121.3±32.9 |
| 500mg/kg | 5mg/kg | 10mg/kg | n=6 | 6.7±16.5** | 14.5±35.6* | 21.3±52.1* |

Each value in the table is shown as that in the Example 1, and "*" denotes a case in which the value was significant versus that of the group treated with thioperamide and metoprine concomitantly, i.e. the group without metoprine administration, by p<0.05, while "**" denotes a case in which the value was significant by p<0.01.

According to the results, in a case when histidine was not administered, effects were not found, although thioperamide and metoprine were concomitantly used. However, a marked effect was obtained by using these three drugs, even when the dose of histidine was 500 mg/kg. Considering the results as well as the results of the above Examples 1 and 2, administration of histidine alone in a single dosage does not provide a suppressive effect on cerebral infarction, but a single dose has been found to show a suppressive effect by administering H₃-receptor blocker or HMT inhibitor concomitantly. Furthermore, an effect was obtained by a using the three drugs concomitantly, even when the dose of histidine itself was reduced.

From the results, it was demonstrated that in the present invention a high suppressive effect on cerebral infarction could be obtained by a use of the three drugs of histidine, H₃-receptor blocker and HMT inhibitor concomitantly.

### Example 4

One of the causes for organ dysfunction resulting from ischemia is reperfusion injury after recovery of blood flow, and the inflammatory response is an important factor in reperfusion injury. Therefore, as an index of inflammation subsequent to reperfusion of blood flow, the numbers of neutrophils and macrophages were determined.

Thirty-two male Wistar rats weighting about 300 g were divided into 4 groups having 8 rats in each. The middle cerebral artery of all rats was occluded for two hours, as shown in Example 1. Physiological saline was given twice to the two control groups shortly and 6 hours after reperfusion as described in the Example 1. The other two groups were intraperitoneally injected with 1000 mg/kg of histidine and 5 mg/kg of thioperamide shortly after reperfusion and 1000 mg/kg of histidine 6 hours after reperfusion. Subsequently, 12 or 24 hours after recovery of blood flow, the brain was taken out after perfusion with physiological saline. Frozen sections having 6 µm thick were prepared at a distance of about 1.7 mm rostral to the bregma, i.e. anterior fontanel, 0.7 mm rostral to the bregma toward the rostral side, and 0.3 mm caudal to the bregma.

Among the obtained frozen sections, frozen sections from the control animals whose brains were perfused 12 and 24 hours after reperfusion and frozen sections obtained from animals treated with histidine and thioperamide (a total of 4 groups) were subjected to immunohistochemistry with an antibody for myeloperoxidase, which is a marker of neutrophils. After observing these frozen sections by light microscopy, the total numbers of neutrophils on the ischemic and non-ischemic sides were obtained separately. The results are shown in Table 4. Also, frozen sections obtained from identical rats were subjected to immunochemistry with an antibody for ED1, which is a cell surface marker of macrophages. ED1 is considered to exist in cells besides macrophages, and in fact, ED1-positive cells were also observed on the non-ischemic side in the experiment. However, as in Table 2, neutrophils were not observed on non-ischemic side, where inflammation was not observed. Therefore, with regard to ED1-positive cells, the difference in the number of cells between the ischemic and non-ischemic sides was calculated. The difference was determined as the number of macrophages and compared between corresponding groups. The results are shown in Table 5. In Tables 4 and 5, "*" denotes a case in which a significant difference versus the control group was observed by p<0.05 by t-test, and "**" denotes a case in which a significant difference was observed by p<0.01.

### Table 4

**The number of neutrophils 12 hours after onset of reperfusion**

| | | +1.7 mm to the bregma | +0.7 mm to the bregma | -0.3 mm to the bregma |
|---|---|---|---|---|
| Control | Non-ischemic side | 0±0 | 0±0 | 0±0 |
| | Ischemic side | 196±76 | 247±137 | 201±116 |
| Histidine + Thioperamide administration | Non-ischemic side | 2±4 | 3±6 | 0±0 |
| | Ischemic side | 47±55** | 72±86** | 84±81* |

**The number of neutrophils 24 hours after onset of reperfusion**

| | | | | |
|---|---|---|---|---|
| | | +1.7 mm to the bregma | +0.7 mm to the bregma | -0.3 mm to the bregma |
| Control | Non-ischemic side | 0±0 | 0±0 | 0±0 |
| | Ischemic side | 225±114 | 253±80 | 277±111 |
| Histidine + Thioperamide administration | Non-ischemic side | 0±1 | 1±1 | 0±0 |
| | Ischemic side | 143±60 | 174±36* | 163±78* |

As shown in Table 4, neutrophil infiltration was observed on the ischemic side 12 hours and 24 hours after ischemia for 2 hours. On the other hand, neutrophil infiltration was significantly suppressed 12 hours and 24 hours after ischemia in the group treated with histidine and thioperamide concomitantly.

### Table 5

**The number of ED1-positive cells 12 hours after the onset of reperfusion**

| | | +1.7 mm to the bregma | +0.7 mm to the bregma | -0.3 mm to the bregma |
|---|---|---|---|---|
| Control | Non-ischemic side | 194±54 | 263±85 | 245±39 |
| | Ischemic side | 362±140 | 522±241 | 297±96 |
| | Differences | 168±119 | 259±169 | 74±74 |
| Histidine + Thioperamide administration | Non-ischemic side | 186±52 | 215±45 | 229±48 |
| | Ischemic side | 289±82 | 265±38** | 284±63 |
| | Differences | 102±63 | 57±46** | 67±82 |

**The number of ED1-positive cells 24 hours after the onset of reperfusion**

| | | +1.7 mm to the bregma | +0.7 mm to the bregma | -0.3 mm to the bregma |
|---|---|---|---|---|
| Control | Non-ischemic side | 116±26 | 141±50 | 195±61 |
| | Ischemic side | 371±101 | 419±126 | 509±206 |
| | Differences | 255±102 | 279±137 | 315±170 |
| Histidine + Thioperamide administration | Non-ischemic side | 142±49 | 151±38 | 176±20 |
| | Ischemic side | 391±157 | 330±97 | 382±90 |
| | Differences | 250±146 | 178±89 | 206±75 |

As shown in Table 5, 12 and 24 hours after ischemia for 2 hours, ED-1 positive cells were found on both the ischemic and non-ischemic sides, and the number of ED-1 positive cells was greater on the ischemic side than on the non-ischemic side. When observed 12 hours after ischemia, the difference in the number of ED-1 positive cells between the ischemic and non-ischemic sides, which was estimated to be the number of macrophages, significantly decreased in the group treated with histidine and thioperamide concomitantly in the cross section at +0.7 mm rostal to the bregma.

From the above results, neutrophil and macrophage infiltration in the brain after long-time ischemia was demonstrated to be suppressed by administration of histidine, resulting in suppression of inflammatory responses after ischemia.

### INDUSTRIAL APPLICABILITY

The cerebral infarction suppressant of the present invention has fewer side effects, and can effectively suppress brain tissue necrosis attributed to long-time ischemia resulting from cerebral thrombosis, cerebral embolism or the like. Accordingly, the cerebral infarction suppressant of the present invention is extremely useful as a suppressant capable of decreasing cerebral infarction whose effective treatment methods have not been available thus far.

## Claims

1. A cerebral infarction suppressant **characterized in** comprising a histidine and an H₃-receptor blocker as active ingredients.

2. The cerebral infarction suppressant according to claim 1, wherein the H₃-receptor blocker is thioperamide.

3. The cerebral infarction suppressant according to claim 1 or 2, further comprising a histamine N-methyltransferase inhibitor an active ingredient.

4. The cerebral infarction suppressant according to claim 3, wherein the histamine N-methyltransferase inhibitor is metoprine.

5. A cerebral infarction suppressant **characterized in** comprising a histidine and a histamine N-methyltransferase inhibitor as active ingredients.

6. The cerebral infarction suppressant according to claim 5, wherein the histamine N-methyltransferase inhibitor is metoprine.

7. The cerebral infarction suppressant according to any one of claims 1 to 6, for a purpose of suppressing cerebral infarction attributed to long-time ischemia prolonged for not less than 1 hour.

8. The cerebral infarction suppressant according to any one of claims 1 to 6, administered during ischemia-reperfusion or after ischemia-reperfusion.

9. The cerebral infarction suppressant according to any one of claims 1 to 6, administered shortly after ischemia-reperfusion.

10. The cerebral infarction suppressant according to any one of claims 1 to 9, administered a plurality of times.

11. The cerebral infarction suppressant according to claim 10, administered either during ischemia-reperfusion or shortly after ischemia-reperfusion and 4 to 8 hours thereafter.

12. The cerebral infarction suppressant according to claim 10, administered either during ischemia-reperfusion or shortly after ischemia-reperfusion and two or more times every 4 to 8 hours thereafter.

13. The cerebral infarction suppressant according to any one of claims 1 to 9, administered in a continuous manner.

14. A use of a histidine and an H₃-receptor blocker for manufacturing a therapeutic agent for cerebral infarction attributed to long-time ischemia.

15. A use of a histidine, an H₃-receptor blocker and a histamine N-methyltransferase inhibitor for manufacturing a therapeutic agent for cerebral infarction attributed to long-time ischemia.

16. A use of a histidine and a histamine N-methyltransferase inhibitor for manufacturing a therapeutic agent for cerebral infarction attributed to long-time ischemia.

17. The use according to any one of claims 14 to 16, for manufacturing the therapeutic agent for cerebral infarction attributed to long-time ischemia prolonged for not less than 1 hour.

18. The use according to any one of claims 14 to 1.6, for manufacturing the therapeutic agent to be administered during ischemia-reperfusion or after ischemia-reperfusion.

19. The use according to any one of claims 14 to 16, for manufacturing the therapeutic agent to be administered shortly after ischemia-reperfusion.

20. The use according to any one of claims 14 to 19, for manufacturing the therapeutic agent to be administered a plurality of times.

21. The use according to claim 20, for manufacturing the therapeutic agent to be administered either during ischemia-reperfusion or shortly after ischemia-reperfusion and 4 to 8 hours thereafter.

22. The use according to claim 20, for manufacturing the therapeutic agent to be administered either during ischemia-reperfusion or shortly after ischemia-reperfusion and two or more times every 4 to 8 hours thereafter.

23. The use according to any one of claims 14 to 19, for manufacturing the therapeutic agent to be administered in a continuous manner.

24. A method for treating cerebral infarction, comprising:
a step of administering a histidine and an H₃-receptor blocker;
wherein the cerebral infarction is attributed to long-time ischemia.

25. A method for treating cerebral infarction, comprising:
a step of administering a histidine, an H₃-receptor blocker and a histamine N-methyltransferase inhibitor;
wherein the cerebral infarction is attributed to long-time ischemia.

26. A method for treating cerebral infarction, comprising:
a step of administering a histidine and a histamine N-methyltransferase inhibitor;
wherein the cerebral infarction is attributed to long-time ischemia.

27. The method according to any one of claims 24 to 26, for treating cerebral infarction attributed to long-time ischemia prolonged for not less than 1 hour.

28. The method of treatment according to any one of claims 24 to 27, wherein the drugs are administered during ischemia-reperfusion or after ischemia-reperfusion.

29. The method of treatment according to any one of claims 24 to 28, wherein the histidine is administered shortly after ischemia-reperfusion.

30. The method of treatment according to any one of claims 24 to 29, wherein the histidine is administered a plurality of times.

31. The method of treatment according to claim 30, wherein the histidine is administered either during ischemia-reperfusion or shortly after ischemia-reperfusion and 4 to 8 hours thereafter.

32. The cerebral infarction suppressant according to claim 30, administered either during ischemia-reperfusion or shortly after ischemia-reperfusion and two or more times every 4 to 8 hours thereafter.

33. The method of treatment according to any one of claims 24 to 29, wherein the drugs are administered in a continuous manner.
